# EUROPEAN PATENT APPLICATION

(11) **EP 4 316 458 A1**
(43) Date of publication of application: **07.02.2024**
(21) Application number: 22780119.8
(22) Date of filing: 16.03.2022
(51) Int. Cl.: A61K 8/55, A61Q 1/00

(54) **COSMETIC BASE AND COSMETIC COMPRISING PHOSPHORYLCHOLINE-LIKE GROUP-CONTAINING COMPOUND**

(30) Priority: 30.03.2021 JP 2021058564
(71) Applicant: NOF Corporation, Shibuya-ku Tokyo 150-6019 (JP)
(72) Inventor: HARA, Yusuke, Kawasaki-shi, Kanagawa 210-0865 (JP); SEKIGUCHI, Koji, Kawasaki-shi, Kanagawa 210-0865 (JP); SASAKI, Katsuyuki, Tokyo 104-0061 (JP); KOIE, Nobuyoshi, Tokyo 104-0061 (JP)
(74) Representative: Müller-Boré & Partner Patentanwälte PartG mbB
(86) International application number: PCT/JP2022/011953
(87) International publication number: WO 2022/209899

(57) **Abstract**

A cosmetic base according to the present invention contains a phosphorylcholine-like group-containing compound represented by Formula (1): 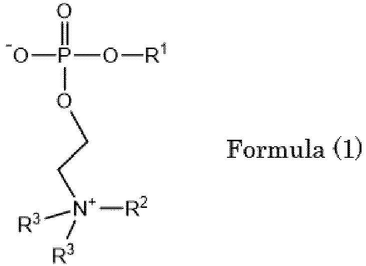 in which each of R¹ and R² indicates a hydrocarbon group having 12 to 22 carbon atoms independently and R³ indicates CH₃ or (CH₂CH₂O)ₙ-H, in which n is an integer of 1 to 30, and either one of R¹ and R² is an unsaturated hydrocarbon group having 18 carbon atoms.

According to the present invention, a novel compound having a phosphorylcholine-like group in its molecule and a cosmetic containing the compound can be provided, the compound being excellent in solubility in oily components and, when blended with cosmetics, retaining emulsion stability at high temperature, providing a crush-feeling of oil drops during applying, and also having a favorable feeling without stiff film-feeling.

## Description

### Technical Field

The present invention relates to a cosmetic base containing a phosphorylcholine-like group-containing compound, and a cosmetic obtained by blending the cosmetic base.

### Background Art

Gemini type surfactants are multi-chain/multi-hydrophilic group-type amphiphilic substances having plural hydrophobic chains and hydrophilic groups in one molecule. Advantages of the gemini type surfactants include (i) high surface-active property represented by low critical micelle concentration and low Krafft point, (ii) high molecule association that enables easy forming of higher order structures such as vesicles, and (iii) favorable washing performance such as antibacterial property, wettability, anti-foamability, anti-metal ion property, and permeability. Because of these useful functions, the gemini type surfactants have widespread use in various industrial fields, including cosmetics industry.

Meanwhile, phosphorylcholine group is an amphoteric polar group having a high hydrophilicity, and representative cosmetic raw materials having the phosphorylcholine group include phospholipids and lecithin. Gemini type surfactants having a similar structure derived from these natural products have been developed so far.

For example, NPL 1 reports a synthesis of a gemini type surfactant having a phosphorylcholine-like group as a hydrophilic group and its solution property, and discloses that the surfactant has an excellent interfacial property.

PTL 1 and PTL 2 report that gemini type compounds having a phosphorylcholine-like group have a high washing power, or a high percutaneous absorption-accelerating property and a moisture retainability-enhancing effect indicating an excellent barrier function or the like.

However, all of hereby-disclosed gemini type surfactants have a hydrocarbon group as the hydrophobic chain. When the chain is long, the compounds are poor in solubility in water or oily components and therefore, there were problems that they were hard to be blended with cosmetics, and that after applying the cosmetics blended with these compounds on skin, stiff film-feeling occurred. On the other hand, when the hydrophobic chain is short, solubility in water or oil improves but is still insufficient, and there was a problem that cosmetics blended with these compounds were poor in stability at high temperature.

PTLs 3 to 5 propose compounds having a favorable water solubility and surface-active property, and having an excellent feeling in use, even with a long-chain hydrocarbon group, by introducing an amide bond having a high hydrophilicity into a part of the hydrophobic chain of the compounds having a phosphorylcholine-like structure. However, since the amide group in these compounds has an excessively high hydrophilicity, there were problems that emulsion stability at high temperature was insufficient when the compounds were used as a cosmetic base and that the stiff film-feeling still occurred after applying when the compounds were blended with cosmetics.

### Citation List

### Patent Literature

PTL 1: JP 2001-262184 A
PTL 2: JP 2011-213602 A
PTL 3: JP 2012-201617 A
PTL 4: JP 2013-1644A
PTL 5: JP 2013-1645 A

### Non Patent Literature

NPL 1: Org. Lett., Vol. 1, No. 9, pp. 1347-1350 (1999)

### Summary of Invention

### Technical Problem

An object of the present invention is to provide a novel compound having a phosphorylcholine-like group in its molecule and a cosmetic containing the compound, the compound being excellent in solubility in oily components and, when blended with cosmetics, retaining emulsion stability at high temperature, providing a crush-feeling of oil drops during applying, and also having a favorable feeling without stiff film-feeling.

### Solution to Problem

As a result of diligent studies to solve the problems described above, the present inventors have found that a cosmetic base containing a phosphorylcholine-like group-containing compound having a specific structure is excellent in solubility in oily components, emulsion stability at high temperature improves remarkably when the cosmetic base is blended with a cosmetic, and the cosmetic provides crush-feeling of oil drops during applying and exhibits a favorable feeling in use without stiff film-feeling, whereby completing the present invention.

That is, the present invention is as indicated below.
[1] A cosmetic base containing a phosphorylcholine-like group-containing compound represented by Formula (1): 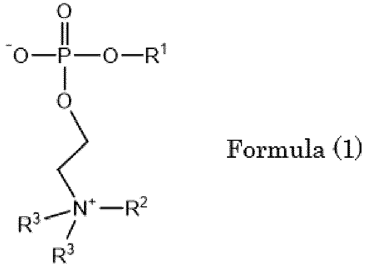 in which each of R¹ and R² indicates a hydrocarbon group having 12 to 22 carbon atoms independently and R³ indicates CH₃ or (CH₂CH₂O)ₙ-H, in which n is an integer of 1 to 30, and either one of R¹ and R² is an unsaturated hydrocarbon group having 18 carbon atoms.
[2] A cosmetic obtained by blending the cosmetic base according to the above [1].

### Advantageous Effects of Invention

The cosmetic base containing a phosphorylcholine-like group-containing compound according to the present invention is excellent in solubility in oily components, retains emulsion stability at high temperature when blended with cosmetics, provides crush-feeling of oil drops during applying when blended with cosmetics, and also has a favorable feeling without stiff film-feeling. Therefore, the cosmetic base is useful as skin cosmetics and hair cosmetics.

### Description of Embodiments

The present invention will be explained in more detail below.

### [Cosmetic Base]

The cosmetic base of the present invention contains a phosphorylcholine-like group-containing compound represented by Formula (1). Hereinafter, the phosphorylcholine-like group-containing compound represented by Formula (1) may be described simply as "PC compound".

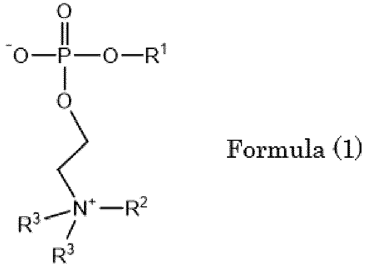

In Formula (1), each of R¹ and R² indicates a hydrocarbon group having 12 to 22 carbon atoms independently and R³ indicates CH₃ or (CH₂CH₂O)ₙ-H, in which n is an integer of 1 to 30, and either one of R¹ and R² is an unsaturated hydrocarbon group having 18 carbon atoms.

In Formula (1), each of R¹ and R² is a hydrocarbon group having 12 to 22 carbon atoms independently. The hydrocarbon group having 12 to 22 carbon atoms is a saturated hydrocarbon group or an unsaturated hydrocarbon group, and may be linear, branched, or cyclic hydrocarbon group. Note that either one of R¹ and R² is an unsaturated hydrocarbon group having 18 carbon atoms. The other one of R¹ and R² is not an unsaturated hydrocarbon group.

Preferably, either one of R¹ and R² is an unsaturated hydrocarbon group having 18 carbon atoms and the other one is a saturated hydrocarbon group having 12 to 22 carbon atoms. More preferably, R¹ is a saturated hydrocarbon group having 12 to 22 carbon atoms and R² is an unsaturated hydrocarbon group having 18 carbon atoms. Especially preferably, R¹ is a saturated hydrocarbon group having 18 to 22 carbon atoms and R² is an unsaturated hydrocarbon group having 18 carbon atoms.

When neither R¹ nor R² is an unsaturated hydrocarbon group, stiff film-feeling is expressed when the PC compound is blended with cosmetics, and crush-feeling of oil drops cannot be provided during applying. When both of R¹ and R² are unsaturated hydrocarbon groups, emulsion stability at high temperature is poor. When R¹ and/or R² has less than 12 carbon atoms, emulsion stability at high temperature deteriorates when the PC compound is used for cosmetics or the like. On the other hand, when R¹ and/or R² has more than 22 carbon atoms, stiff film-feeling is expressed when the PC compound is blended with cosmetics.

Examples of the saturated hydrocarbon group having 12 to 22 carbon atoms include lauryl group, stearyl group, and behenyl group. The saturated hydrocarbon group having 12 to 22 carbon atoms is preferably stearyl group or behenyl group, and more preferably behenyl group.

The unsaturated hydrocarbon group having 18 carbon atoms is preferably oleyl group.

In Formula (1), R³ indicates CH₃ or (CH₂CH₂O)ₙ-H, and n is an integer of 1 to 30. Especially from viewpoints of emulsion stability and long-lasting moisture retainability after drying, preferably n is an integer of 1 to 5, more preferably n is an integer of 1 to 3, and especially preferably n is 1, that is, R³ is CH₂CH₂OH.

### [Production Method of PC Compound]

The PC compound used in the present invention can be obtained by performing a reaction between an alcohol represented by Formula (2) and 2-chloro-2-oxo-1,3,2-dioxaphosphorane (hereinafter abbreviated as COP) in the presence of an organic base to obtain an intermediate, and ring-opening the intermediate with a tertiary amine represented by Formula (3). The obtained PC compound can be purified by a general purification method such as a reprecipitation or a recrystallization.

R¹-OH (2)

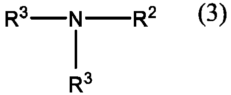

In Formula (2) and Formula (3), R¹, R², and R³ mean the same as in Formula (1).

### [Cosmetic]

Forms of the cosmetic obtained by blending the cosmetic base according to the present invention are not particularly limited and can be any of aqueous cosmetics, water-in-oil type or oil-in-water type emulsified cosmetics, and oily cosmetics. Examples of the forms include hair cosmetics such as shampoo, rinse, hair mist, hair cream, and hair foam, skincare cosmetics and make-up cosmetics such as skin lotion, milky lotion, cream, essence lotion, and foundation, massage cosmetics, and pack cosmetics. The cosmetic may be various forms including body care cosmetics such as bath products, body shampoo, and hand soap.

The cosmetic according to the present invention contains a cosmetic material and the PC compound. The blending proportion of the PC compound is not particularly limited, and normally, the PC compound is blended at 0.001 to 20% by mass relative to the total of the cosmetic. When the blending proportion is less than 0.001% by mass, desired effects may be hard to be obtained since the amount of the PC compound is insufficient. When the blending proportion is more than 20% by mass, handleability during preparing the cosmetic may deteriorate and the obtained cosmetic may not provide a favorable feeling in use.

The cosmetic according to the present invention may contain other components as far as the performance of the present invention is not impaired. Examples of the other components include lower alcohols, hydrocarbon oils, natural oils and fats, synthetic triglycerides, ester oils, waxes, silicone derivatives, oil base agents, anionic surfactants, amphoteric surfactants, nonionic surfactants, cationic surfactants, semipolar surfactants, water soluble polymers, organic or inorganic salts, pH regulators, bactericides, chelating agents, antioxidants, UV absorbers, vitamins, natural extracts from animals and plants, dyes, pigments, and fragrances.

### Examples

The present invention will be explained below in more detail with reference to Examples and Comparative Examples, but the present invention is not limited thereto.

### (NMR Measurement)

PC compound obtained in accordance with Synthesis Examples was dissolved in heavy chloroform containing tetramethylsilane (TMS) as an internal standard substance, and then the compound was analyzed using JNM-AL600 (manufactured by JEOL Ltd.).

### [Synthesis Example 1: (Synthesis of PC1)]

Into a 1 L-round flask equipped with a thermometer, a dropping funnel, and a stirrer were added 53.6 g (0.2 mol) of oleyl alcohol as the raw material alcohol, 20.2 g (0.2 mol) of triethylamine, and 280 g of tetrahydrafuran, followed by cooling to 4°C, stirring and mixing. Next, a mixture solution of 28.5 g (0.2 mol) of 2-chloro-2-oxo-1,3,2-dioxaphosphorane and 60 g of tetrahydrafuran was added dropwisely to the above-described cooled mixture solution using the dropping funnel. The dropwise addition was performed gradually over two hours while stirring the cooled mixture solution and performing cooling so that the reaction temperature did not exceed 10°C. After the completion of the dropwise addition, stirring was continued for one more hour. Then, triethylamine hydrochloride precipitated as a by-product was filtered out. The whole amount of filtrate obtained was charged into a 2 L round-bottom flask equipped with a stirrer, and 85.3 g (0.4 mol) of N,N-dimethyllaurylamine as the tertiary amine and 380 g of acetonitrile were added thereto, followed by stirring at 70°C for 12 hours. Thereafter, precipitate obtained by cooling the reaction solution was filtered, and vacuum dried at 70°C to obtain 19.1 g of crude crystal. The obtained crude crystal was recrystallized in a mixture solvent of tetrahydrafuran and acetonitrile to obtain 21.1 g of white crystal (yield: 18%). The analysis results of ¹H-NMR and ³¹P-NMR of the obtained PC1 are indicated below.
¹H-NMR (δ (ppm)): 0.88 (t, J (HH) = 7.2 Hz, 6H), 1.25-1.4 (m, 40H), 1.57-1.63 (m, 2H), 1.68-1.72 (m, 2H), 1.95-2.02 (m, 4H), 3.33 (s, 6H), 3.36-3.46 (m, 2H), 3.78-3.84 (m, 4H), 4.28-4.32 (m, 2H), 5.31-5.37 (m, 2H)
³¹P-NMR (δ (ppm)): 0.77 (s)

### [Synthesis Example 2: (Synthesis of PC2)]

The same operations as in Synthesis Example 1 were performed except for using 141.4 g (0.4 mol) of N,N-dimethylbehenylamine instead of N,N-dimethyllaurylamine as the raw material amine to obtain 43.6 of white crystal (yield: 30%). The analysis results of ¹H-NMR and ³¹P-NMR of the obtained PC2 are indicated below.
¹H-NMR (δ (ppm)): 0.88 (t, J (HH) = 7.2 Hz, 6H), 1.3-1.5 (m, 60H), 1.5-1.6 (m, 2H), 1.65-1.7 (m, 2H), 1.95-2.02 (m, 4H), 3.3 (s, 6H), 3.3-3.46 (m, 2H), 3.78-3.84 (m, 4H), 4.27-4.31 (m, 2H), 5.31-5.38 (m, 2H)
³¹P-NMR (δ (ppm)): 0.56 (s)

### [Synthesis Example 3: (Synthesis of PC3)]

The same operations as in Synthesis Example 1 were performed except for using 54.1 g (0.2 mol) of stearyl alcohol instead of oleyl alcohol as the raw material alcohol, and 118.2 g (0.4 mol) of N,N-dimethyloleylamine instead of N,N-dimethyllaurylamine as the raw material amine to obtain 40.3 g of white crystal (yield: 30%). The analysis results of ¹H-NMR and ³¹P-NMR of the obtained PC3 are indicated below.
¹H-NMR (δ (ppm)): 0.88 (t, J (HH) = 7.2 Hz, 6H), 1.28-1.4 (m, 52H), 1.56-1.62 (m, 2H), 1.65-1.7 (m, 2H), 1.93-2.02 (m, 4H), 3.34 (s, 6H), 3.43-3.47 (m, 2H), 3.76-3.84 (m, 4H), 4.28-4.31 (m, 2H), 5.3-5.4 (m, 2H)
³¹P-NMR (δ (ppm)): 0.48 (s)

### [Synthesis Example 4: (Synthesis of PC4)]

The same operations as in Synthesis Example 1 were performed except for using 65.3 g (0.2 mol) of behenyl alcohol instead of oleyl alcohol as the raw material alcohol, and 118.2 g (0.4 mol) of N,N-dimethyloleylamine instead of N,N-dimethyllaurylamine as the raw material amine to obtain 72.8 g of white crystal (yield: 50%). The analysis results of ¹H-NMR and ³¹P-NMR of the obtained PC4 are indicated below.
¹H-NMR (δ (ppm)): 0.88 (t, J (HH) = 7.2 Hz, 6H), 1.2-1.34 (m, 48H), 1.54-1.61 (m, 2H), 1.63-1.7 (m, 2H), 1.93-2.09 (m, 4H), 3.36 (s, 6H), 3.43-3.47 (m, 2H), 3.78-3.87 (m, 4H), 4.29-4.32 (m, 2H), 5.3-5.4 (m, 2H)
³¹P-NMR (δ (ppm)): 0.39 (s)

### [Synthesis Example 5: (Synthesis of PC5)]

The same operations as in Synthesis Example 1 were performed except for using 65.3 g (0.2 mol) of behenyl alcohol instead of oleyl alcohol as the raw material alcohol, and 142 g (0.4 mol) of N,N-diethanololeylamine instead of N,N-dimethyllaurylamine as the raw material amine to obtain 15.7 g of white crystal (yield: 10%). The analysis results of ¹H-NMR and ³¹P-NMR of the obtained PC5 are indicated below.
¹H-NMR (δ (ppm)): 0.88 (t, J (HH) = 6.6 Hz, 6H), 1.21-1.39 (m, 60H), 1.58-1.63 (m, 2H), 1.65-1.73 (m, 2H), 1.93-2.1 (m, 4H), 3.6-3.65 (m, 4H), 3.7-3.75 (m, 2H), 3.8-3.85 (m, 4H), 3.95-4.05 (m, 4H), 4.51-4.55 (m, 2H), 5.3-5.4 (m, 2H)
³¹P-NMR (δ (ppm)): -2.1 (s)

### [Synthesis Example 6: (Synthesis of PC6)]

The same operations as in Synthesis Example 1 were performed except for using 165.4 g (0.4 mol) of N,N-diethanolbehenylamine instead of N,N-dimethyllaurylamine as the raw material amine to obtain 15.5 g of white crystal (yield: 10%). The analysis results of ¹H-NMR and ³¹P-NMR of the obtained PC6 are indicated below.
¹H-NMR (δ (ppm)): 0.88 (t, J (HH) = 6.6 Hz, 6H), 1.2-1.38 (m, 64H), 1.58-1.64 (m, 2H), 1.67-1.72 (m, 2H), 1.93-2.09 (m, 4H), 3.7-3.76 (m, 4H), 3.83-3.86 (m, 2H), 3.92-3.96 (m, 4H), 4.0-4.05 (m, 2H), 4.17-4.25 (m, 4H), 5.3-5.4 (m, 2H)
³¹P-NMR (δ (ppm)): -1.1 (s)

### [Comparative Synthesis Example 1: (Synthesis of PC7)]

The same operations as in Synthesis Example 1 were performed except for using 54.1 g (0.2 mol) of stearyl alcohol instead of oleyl alcohol as the raw material alcohol to obtain 35.3 g of white crystal (yield: 33%). The analysis results of ¹H-NMR and ³¹P-NMR of the obtained PC7 are indicated below.
¹H-NMR (δ (ppm)): 0.88 (t, J (HH) = 7.2 Hz, 6H), 1.24-1.38 (m, 48H), 1.58-1.64 (m, 2H), 1.7-1.75 (m, 2H), 3.15 (s, 6H), 3.4-3.47 (m, 2H), 3.53-3.58 (m, 2H), 3.82-3.88 (m, 2H), 4.19-4.25 (m, 2H)
³¹P-NMR (δ (ppm)): 0.56 (s)

### [Comparative Synthesis Example 2: (Synthesis of PC8)]

The same operations as in Synthesis Example 1 were performed except for using 54.1 g (0.2 mol) of stearyl alcohol instead of oleyl alcohol as the raw material alcohol, and g (0.4 mol) of N,N-diethanollaurylamine instead of N,N-dimethyllaurylamine as the raw material amine to obtain 72.6 g of white crystal (yield: 50%). The analysis results of ¹H-NMR and ³¹P-NMR of the obtained PC8 are indicated below.
¹H-NMR (δ (ppm)): 0.88 (t, J (HH) = 6.6 Hz, 6H), 1.22-1.38 (m, 52H), 1.58-1.7 (m, 4H), 3.38-3.5 (m, 4H), 3.71-3.77 (m, 2H), 3.81-3.86 (m, 2H), 3.89-4.05 (m, 4H), 4.12-4.19 (m, 2H)
³¹P-NMR (δ (ppm)): 1.6 (s)

### [Comparative Synthesis Example 3: (Synthesis of PC9)]

The same operations as in Synthesis Example 1 were performed except for using 54.1 g (0.2 mol) of isostearyl alcohol instead of oleyl alcohol as the raw material alcohol to obtain 23.5 g of white crystal (yield: 20%). The analysis results of ¹H-NMR and ³¹P-NMR of the obtained PC9 are indicated below.
¹H-NMR (δ (ppm)): 0.71-0.89 (m, 9H), 1.21-1.35 (m, 49H), 1.55-1.63 (m, 2H), 1.65-1.71 (m, 2H), 3.2 (s, 6H), 3.35-3.37 (m, 2H), 3.65-3.69 (m, 2H), 3.78-3.82 (m, 2H), 4.22-4.24 (m, 2H)
³¹P-NMR (δ (ppm)): 0.56 (s)

### [Comparative Synthesis Example 4: (Synthesis of PC10)]

The same operations as in Synthesis Example 1 were performed except for using 54.1 g (0.2 mol) of stearyl alcohol instead of oleyl alcohol as the raw material alcohol, and 141.4 g (0.4 mol) of N,N-dimethylbehenylamine as the raw material amine to obtain 26.1 g of white crystal (yield: 17.9%). The analysis results of ¹H-NMR and ³¹P-NMR of the obtained PC10 are indicated below.
¹H-NMR (δ (ppm)): ppm)): 0.88 (6H, m), 1.27 (60H, m), 1.61 (2H, m), 1.75 (2H, m), 3.18 (6H, s), 3.42 (2H, m), 3.66 (2H, m), 3.83 (2H, t), 4.26 (2H, t)
³¹P-NMR (δ (ppm)): 2.76 (s)

### [Comparative Synthesis Example 5: (Synthesis of PC11)]

The same operations as in Synthesis Example 1 were performed except for using 118.2 g (0.4 mol) of N,N-dimethyloleylamine instead of N,N-dimethyllaurylamine as the raw material amine to obtain 16.08 g of white crystal (yield: 12%). The analysis results of ¹H-NMR and ³¹P-NMR of the obtained PC11 are indicated below.
¹H-NMR (δ (ppm)): 0.88 (t, J (HH) = 7.2 Hz, 6H), 1.2-1.4 (m, 48H), 1.56-1.62 (m, 2H), 1.65-1.7 (m, 2H), 1.93-2.05 (m, 4H), 3.34 (s, 6H), 3.44-3.47 (m, 2H), 3.76-3.84 (m, 4H), 4.28-4.31 (m, 2H), 5.3-5.4 (m, 4H)
³¹P-NMR (δ (ppm)): 0.56 (s)

### [Comparative Synthesis Example 6: (Synthesis of PC12)]

The same operations as in Synthesis Example 1 were performed except for using 54.1 g (0.2 mol) of stearyl alcohol instead of oleyl alcohol as the raw material alcohol, and 147.5 g (0.4 mol) of stearic acid dimethylaminopropylamide instead of N,N-dimethyllaurylamine as the raw material amine to obtain 20.5 g of white crystal (yield: 20%). The analysis results of ¹H-NMR and ³¹P-NMR of the obtained PC 12 are indicated below.
¹H-NMR (δ (ppm)): 0.96 (6H, m), 1.29 (54H, m), 1.33 (4H, m), 1.48 (2H, m), 1.57 (2H, m), 1.99 (2H, m), 2.18 (2H, m), 3.20 (2H, m), 3.24 (2H, m), 3.30 (6H, m), 3.43 (2H, t), 3.53 (2H, m), 3.97 (2H, t), 8.00 (1H, s),
³¹P-NMR (δ (ppm)): 3.76 (s)

Details of the 11 compounds including cosmetic bases of the present invention synthesized in Synthesis Examples 1 to 6 and compounds synthesized in Comparative Synthesis Examples 1 to 5 are indicated in Table 1. The compound synthesized in Comparative Synthesis Example 6 is indicated as Formula (4). R¹, R², and R³ in Table 1 correspond to those in Formula (1).

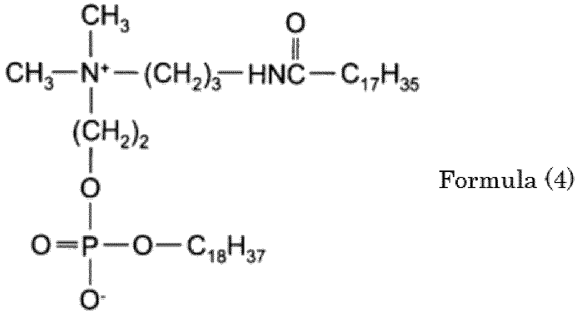

### (Examples 1 to 6, Comparative Examples 1 to 7)

In accordance with the formulations in Table 2, components of oil phase and water phase were each dissolved at 70°C. The oil phase, while being stirred, was added to the water phase, followed by stirring with a homo mixer. Regarding emulsified cosmetic obtained, results of evaluating uniformity of the oil phase during production and emulsion stability at high temperature based on the criteria below, and performing sensory evaluations below are indicated in Table 3. Note that the values in Table 2 are in parts by mass.

### (Uniformity of Oil Phase)

A: Uniformly dissolved
B: Slight turbidness is seen
C: Insoluble matter partly exists
D: Insoluble matter exists

### (Emulsion Stability at High Temperature)

The emulsified cosmetic obtained was stored at 50°C for one month and then the appearance thereof was evaluated based on the criteria below.
A: Uniform
B: Slight separation is seen
C: Partial separation is seen
D: Separated in two layers

### (Sensory Evaluation)

Appropriate amount of the emulsified cosmetic obtained was applied on an inner part of the evaluator's forearm and (1) absence of stiff film-feeling after applying, (2) crush-feeling of oil drops during applying, and (3) moisture feeling at 5 hours after applying were evaluated by 5 grades based on the criteria below. Evaluation was performed by 10 people, and the evaluation points were summed up.

### (Sensory Evaluation Criteria)

Evaluation points; 5: very good, 4: good, 3: normal, 2: rather bad, 1: bad

**Table 1**

| | Compound abbreviation | R¹ | R² | R³ |
|---|---|---|---|---|
| Synthesis Example 1 | PC1 | Oleyl group | Lauryl group | Methyl group |
| Synthesis Example 2 | PC2 | Oleyl group | Behenyl group | Methyl group |
| Synthesis Example 3 | PC3 | Stearyl group | Oleyl group | Methyl group |
| Synthesis Example 4 | PC4 | Behenyl group | Oleyl group | Methyl group |
| Synthesis Example 5 | PC5 | Behenyl group | Oleyl group | -CH₂CH₂OH |
| Synthesis Example 6 | PC6 | Oleyl group | Behenyl group | -CH₂CH₂OH |
| Comparative Synthesis Example 1 | PC7 | Stearyl group | Lauryl group | Methyl group |
| Comparative Synthesis Example 2 | PC8 | Stearyl group | Lauryl group | -CH₂CH₂OH |
| Comparative Synthesis Example 3 | PC9 | Isostearyl group | Lauryl group | Methyl group |
| Comparative Synthesis Example 4 | PC10 | Stearyl group | Behenyl group | Methyl group |
| Comparative Synthesis Example 5 | PC11 | Oleyl group | Oleyl group | Methyl group |

**Table 2**

| | | Examples | | | | | | Comparative Examples | | | | | | |
|---|---|---|---|---|---|---|---|---|---|---|---|---|---|---|
| Components Name | | 1 | 2 | 3 | 4 | 5 | 6 | 1 | 2 | 3 | 4 | 5 | 6 | 7 |
| Oil phase | Synthesis Example 1 (PC1) | 1 | - | - | - | - | - | - | - | - | - | - | - | - |
| | Synthesis Example 2 (PC2) | - | 1 | - | - | - | - | - | - | - | - | - | - | - |
| | Synthesis Example 3 (PC3) | - | - | 1 | - | - | - | - | - | - | - | - | - | - |
| | Synthesis Example 4 (PC4) | - | - | - | 1 | - | - | - | - | - | - | - | - | - |
| | Synthesis Example 5 (PC5) | - | - | - | - | 1 | - | - | - | - | - | - | - | - |
| | Synthesis Example 6 (PC6) | - | - | - | - | - | 1 | - | - | - | - | - | - | - |
| | Comparative Synthesis Example 1 (PC7) | - | - | - | - | - | - | 1 | | | | - | - | - |
| | Comparative Synthesis Example 2 (PC8) | - | - | - | - | - | - | - | 1 | - | - | - | - | - |
| | Comparative Synthesis Example 3 (PC9) | - | - | - | - | - | - | - | - | 1 | | - | - | - |
| | Comparative Synthesis Example 4 (PC10) | - | - | - | - | - | - | - | - | - | 1 | - | - | - |
| | Comparative Synthesis Example 5 (PC11) | - | - | - | - | - | - | - | - | - | - | 1 | - | - |
| | Comparative Synthesis Example 6 (PC12) | - | - | - | - | - | - | - | - | - | - | - | 1 | - |
| | Hydrogenated lecithin | - | - | - | - | - | - | - | - | - | - | - | - | 1 |
| | Behenyl alcohol | 0.5 | 0.5 | 0.5 | 0.5 | 0.5 | 0.5 | 0.5 | 0.5 | 0.5 | 0.5 | 0.5 | 0.5 | 0.5 |
| | Cetostearyl alcohol | 0.5 | 0.5 | 0.5 | 0.5 | 0.5 | 0.5 | 0.5 | 0.5 | 0.5 | 0.5 | 0.5 | 0.5 | 0.5 |
| | Stearic acid glyceryl | 0.5 | 0.5 | 0.5 | 0.5 | 0.5 | 0.5 | 0.5 | 0.5 | 0.5 | 0.5 | 0.5 | 0.5 | 0.5 |
| | Isostearic acid | 2 | 2 | 2 | 2 | 2 | 2 | 2 | 2 | 2 | 2 | 2 | 2 | 2 |
| | Tri 2-ethylhexanoic acid glyceryl | 2 | 2 | 2 | 2 | 2 | 2 | 2 | 2 | 2 | 2 | 2 | 2 | 2 |
| | Liquid paraffin | 10 | 10 | 10 | 10 | 10 | 10 | 10 | 10 | 10 | 10 | 10 | 10 | 10 |
| | Dimethicone | 0.5 | 0.5 | 0.5 | 0.5 | 0.5 | 0.5 | 0.5 | 0.5 | 0.5 | 0.5 | 0.5 | 0.5 | 0.5 |
| Water phase | Xanthan gum | 0.1 | 0.1 | 0.1 | 0.1 | 0.1 | 0.1 | 0.1 | 0.1 | 0.1 | 0.1 | 0.1 | 0.1 | 0.1 |
| | Phenoxyethanol | 0.3 | 0.3 | 0.3 | 0.3 | 0.3 | 0.3 | 0.3 | 0.3 | 0.3 | 0.3 | 0.3 | 0.3 | 0.3 |
| | Alcohol | 2 | 2 | 2 | 2 | 2 | 2 | 2 | 2 | 2 | 2 | 2 | 2 | 2 |
| | Glycerin | 3 | 3 | 3 | 3 | 3 | 3 | 3 | 3 | 3 | 3 | 3 | 3 | 3 |
| | 1,3-Butylene glycol | 5 | 5 | 5 | 5 | 5 | 5 | 5 | 5 | 5 | 5 | 5 | 5 | 5 |
| | Purified water | 72.6 | 72.6 | 72.6 | 72.6 | 72.6 | 72.6 | 72.6 | 72.6 | 72.6 | 72.6 | 72.6 | 72.6 | 72.6 |
| Total | | 100 | 100 | 100 | 100 | 100 | 100 | 100 | 100 | 100 | 100 | 100 | 100 | 100 |

**Table 3**

| | Examples | | | | | | Comparative Examples | | | | | | |
|---|---|---|---|---|---|---|---|---|---|---|---|---|---|
| Evaluation items | 1 | 2 | 3 | 4 | 5 | 6 | 1 | 2 | 3 | 4 | 5 | 6 | 7 |
| Uniformity of oil phase | B | B | B | A | A | A | C | D | C | D | A | D | A |
| Emulsion stability (50°C, 1 month) | B | B | B | B | A | A | C | C | D | B | D | D | C |
| Absence of stiff film-feeling | 39 | 38 | 45 | 48 | 48 | 39 | 20 | 35 | 30 | 10 | 48 | 10 | 45 |
| Crush-feeling of oil drops during applying | 36 | 43 | 43 | 45 | 45 | 46 | 25 | 28 | 29 | 28 | 45 | 30 | 10 |
| Moisture feeling at 5 hours after applying | 35 | 44 | 40 | 46 | 46 | 45 | 20 | 41 | 35 | 35 | 40 | 31 | 41 |

According to Table 3, it was found that the compounds synthesized in Synthesis Examples 1 to 6 were excellent in solubility in oily components, the emulsified cosmetics blended with those compounds had high emulsion stability at high temperature, and when the emulsified cosmetics were applied on skin, it had a favorable feeling without stiff film-feeling after applying, providing crush-feeling of oil drops during applying, and maintaining a long-term moisture feeling even after applying. When the gemini type compound includes no unsaturated hydrocarbon group as in Comparative Examples 1, 2, 3, and 4, stiff film-feeling is expressed while applying, and favorable feeling such as crush-feeling of oil drops is not expressed. When both hydrocarbon groups of the gemini type compound are unsaturated hydrocarbon as in Comparative Example 5, emulsion stability is poor. In the gemini type compound having an amide group as in Comparative Example 6, solubility in oil phase and emulsion stability are poor, and stiff film-feeling is expressed after applying. In the surfactant having a phosphorylcholine group having a structure different from the present application as in Comparative Example 7, emulsion stability is poor, and favorable feeling such as crush-feeling of oil drops is not expressed during applying.

## Claims

1. A cosmetic base comprising a phosphorylcholine-like group-containing compound represented by Formula (1): 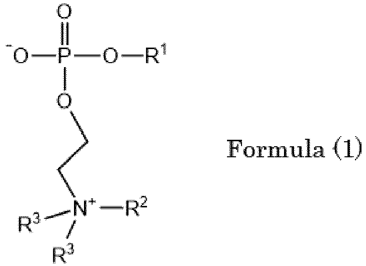 wherein each of R¹ and R² indicates a hydrocarbon group having 12 to 22 carbon atoms independently and R³ indicates CH₃ or (CH₂CH₂O)ₙ-H, wherein n is an integer of 1 to 30, and either one of R¹ and R² is an unsaturated hydrocarbon group having 18 carbon atoms.

2. A cosmetic obtained by blending the cosmetic base according to Claim 1.
